# EUROPEAN PATENT APPLICATION

(11) **EP 2 508 191 A1**
(43) Date of publication of application: **10.10.2012**
(21) Application number: 12156263.1
(22) Date of filing: 15.03.2007
(51) Int. Cl.: A61K 36/481, A61K 36/236, A61K 36/88, A61K 35/56, A61K 36/537, A61P 9/04, A61K 35/62, A61K 36/65, A61P 25/28, A61K 35/64, A61K 36/69, A61K 45/06, A61K 36/258

(54) **Combination therapy for treatment of patients with neurological disorders and cerebral infarction**

(30) Priority: 16.03.2006 US 782798 P
(62) Divisional of application: 07716160.2
(71) Applicant: Moleac Pte Ltd., Singapore 138667 (SG)
(72) Inventor: Shi, Xuemin, 300193 Nankai District (CN); Bousser, Marie Germaine, 75004 Paris (FR); Picard, David, Singapore 138667 (SG)
(74) Representative: Harrison Goddard Foote

(57) **Abstract**

The present invention provides compositions and methods of treating a patient having a condition selected from the group of cerebral stroke, heart stroke, neurodegenerative diseases, brain or nervous system trauma, or neuroplasticity wherein the composition comprises: (i) at least 4, 5, 6, 7, 8, 9, 10, 11, 12, 13 or 14 of: Radix Astragali (milkvetch root or Huang Qi); Rhizoma Salviae Miltiorrhizae root (red sage or Dan Shen); Radix Paeoniae Rubra (red peony or chi shao); Ligusticum Chuanxiong (Chuan Xiong); Rhizoma Notoginseng (Sanqi); Odoriferous Rosewood (Jiang Xiang); Scorpion (Quan Xie); Radix Polygalae (Yuan Zhi); Grassleaf sweetflag (Shi Changpu); Leech (Hirudo or Shuizhi); Ground beetle (Tu Bie Chong); Cow bezoar (calculus Bovis artifactus or Rengong Niuhuang); Gambirplant (ramulus uncariae cum uncis or Gou teng); and (ii) an agent used in Western medicine.

## Description

### FIELD OF THE INVENTION

The present invention provides methods and compositions for the treatment of stroke and neurological disorders. The methods and compositions of the present invention bring together aspects of Traditional Chinese Medicine (TCM) and Western medicine.

### BACKGROUND

Stroke is a major cause of death and disability. Primary stroke prevention focuses on lifestyle modifications of risk factors while secondary stroke prevention aims to reduce the overall risk of recurrence in persons who have had a stroke.

There is currently a lack of treatment methods for stroke recovery in Western medicine, these being frequently limited to the following options:
a) Intra-arterial thrombolysis with intravenous tissue plasminogen activator (rt-PA), which is applicable only to 3 to 5% of stroke patients (as it has to be applied only for acute ischemic forms, only if patients do not present any contra-indication, and only within 3 to 6 hours after the onset of symptoms), can restore blood perfusion and prevent neurological and functional damage to some degree;
b) administration of aspirin / other antiplatelets / or sometimes anticoagulant to most of the cerebral stroke patients for secondary stroke prevention, which gives an improvement effect of about 1% (The International Stroke Trial (IST) a randomised trial of aspirin, subcutaneous heparin, both or neither among 19435 patients with acute ischaemic stroke. International Stroke Trial Collaborative Group. Lancet. 1997 May 31; 349(9065):1569-81);
c) medications such as analgesics may be needed to control associated symptoms
d) rehabilitation includes physical therapy such as physiotherapy, massage, speech therapy, or occupational therapy.

One known TCM product is NeuroAid^{®}. Clinical studies performed in China on compositions the same as NeuroAid^{®} have shown that this natural product combination increases stroke patients' neurological disability recovery and functional outcomes with extremely few side effects or other adverse effects. The composition of NeuroAid^{®} has been approved by and registered with the State Food and Drug Administration (SFDA) and is administered for the treatment of cerebral infarct patients during their recovery at an early or late stage, and the indications in Traditional Chinese Medicine are: to supplement qi and activate blood circulation. It is applied to treat those patients who are suffering from ischemic or hemorrhagic (for the latter, it is at present indicated in late phase only) cerebral infarction of qi deficiency and blood stasis with manifestations of hemiplegia, hemianesthesia, wry mouth, aphasia (inarticulateness) and etc during their channel and collateral convalescent period of ischemia apoplexy, and its efficacy is supported by clinical trial data.

It may be useful for treating other types of stroke than cerebro-vascular stroke such as cardiovascular disease (heart stroke mainly due to coronary artery stroke) as well as other neurological disorders. Neurological disorders are disorders that affect the central nervous system, the peripheral nervous system and the autonomic nervous system such as neurodegenerative diseases (for example, Alzheimer's disease and Parkinson's disease), epilepsy, seizure, demyelinating diseases (for example, multiple sclerosis), cerebral palsy, traumatic injuries to or tumours in the brain, spinal cord and peripheral nerves.

NeuroAid^{®} is capable of use as a Western medicine or a dietary supplement to provide nutrition to healthy individuals as well as patients afflicted with stroke or neurological disorders.

NeuroAid^{®} is typically administered orally (per os) as such or by diluting the capsules in water or via a gastric tube, 3 times each day and 4 capsules each time for a 4-week course of treatment. The duration of treatment is typically 3 months/3 courses, adaptable with regard to the patient's condition.

The use of TCM is, however, particularly challenging for European clinicians because of the lack of guidelines, clinical data and the small number of studies conducted under Western guidelines. Potential interactions between TCM and Western medicine that may lead to adverse side effects are also a major concern among both practitioners of TCM and Western medicine. Of particular concern are the increase or decrease in the effects of a blood thinner such as Warfarin that may lead to either a bleeding episode or formation of a blood clot, and the decrease in the effect of a blood pressure medication that may lead to high blood pressure and a stroke. For example, the potential interaction of the Chinese herb salvia with the Western drug Warfarin leading to excessive blood thinning with bleeding has been well documented with confirmatory laboratory studies. Non-steroidal anti-inflammatory drugs (NSAIDS), in particular aspirin, also have the potential to interact with Chinese herbs and increase bleeding risks. For these, and other reasons, TCM is generally not used in conjunction with Western medicines.

As current treatment options do not address the needs of difficult-to-treat patients with important stroke disabilities, such as hemiparalysis or aphasia, the present invention seeks to combine TCM with established agents for the treatment of stroke patients to present a new therapeutic treatment option for stroke patients.

### SUMMARY OF INVENTION

According to a first aspect, there is provided a method of treating a patient having a condition selected from the group of cerebral stroke, heart stroke, neurodegenerative diseases, brain trauma, nervous system trauma and conditions related to neuroplasticity, the method comprising administering to the patient, (i) a composition which comprises at least 4, 5, 6, 7, 8, 9, 10, 11, 12, 13 or 14 of the following ingredients: Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng); and (ii) an agent used in Western medicine for the treatment of cerebral stroke, heart stroke, neurodegenerative diseases, brain trauma, nervous system trauma or conditions related to neuroplasticity.

According to a second aspect, there is provided the use of: (i) a composition which comprises at least 4, 5, 6, 7, 8, 9, 10, 11, 12, 13 or 14 of the following ingredients: Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng); and (ii) an agent used in Western medicine for the treatment of stroke, in the manufacture of a medicament for use in treating patients with cerebral stroke, heart stroke, neurodegenerative diseases, brain trauma, nervous system trauma or conditions related to neuroplasticity.

According to a third aspect, there is provided the use of: a composition which comprises at least 4, 5, 6, 7, 8, 9, 10, 11, 12, 13 or 14 of the following ingredients: Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng), in the manufacture of a medicament for treating a patient having a condition selected from the group consisting of cerebral stroke, heart stroke, neurodegenerative diseases, brain trauma, nervous system trauma and conditions related to neuroplasticity, where the patient also receives an agent used in Western medicine for the treatment of patients with cerebral stroke, heart stroke, neurodegenerative diseases, brain trauma, nervous system trauma or conditions related to neuroplasticity.

According to a fourth aspect, there is provided the use of an agent used in Western medicine for the treatment of cerebral stroke, heart stroke, neurodegenerative diseases, brain trauma, nervous system trauma or conditions related to neuroplasticity in the manufacture of a medicament for treating a patient having a condition selected from the group consisting of cerebral stroke, heart stroke, neurodegenerative diseases, brain trauma, nervous system trauma and conditions related to neuroplasticity where the patient also receives a composition which comprises at least 4, 5, 6, 7, 8, 9, 10, 11, 12, 13 or 14 of the following ingredients: Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng).

According to a fifth aspect, there is provided a product comprising as a combined preparation:
(i) a composition which comprises at least 4, 5, 6, 7, 8, 9, 10, 11, 12, 13 or 14 of the following ingredients: Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng); and (ii) an agent used in Western medicine for the treatment of stroke, for simultaneous, separate or sequential use in the treatment of patient with a cerebral stroke, heart stroke, neurodegenerative diseases, brain trauma, nervous system trauma or conditions related to neuroplasticity, and optionally instructions for use.

According to a sixth aspect, there is provided a method of treating a patient having a condition selected from the group of cerebral stroke, heart stroke, neurodegenerative diseases, brain trauma, nervous system trauma and conditions related to neuroplasticity, the method comprising administering to the patient, (i) NeuroAid^{®}; and (ii) an agent used in Western medicine for the treatment of cerebral stroke, heart stroke, neurodegenerative diseases, brain trauma, nervous system trauma or conditions related to neuroplasticity.

According to a seventh aspect, there is provided a product comprising as a combined preparation (i) NeuroAid^{®}; and (ii) an agent used in Western medicine for the treatment of stroke, for simultaneous, separate or sequential use as a medicament for the treatment of a patient having a condition selected from the group of cerebral stroke, heart stroke, neurodegenerative diseases, brain trauma, nervous system trauma and conditions related to neuroplasticity, and optionally instructions for use.

According to an eight aspect, there is provided a method of identifying at least one compound for treating at least one of cerebral stroke, heart stroke, neurodegenerative diseases, brain trauma, nervous system trauma or conditions related to neuroplasticity, the method comprising the step of selecting one or more isolated compounds from one or more herbs selected from the group consisting of Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng).

According to a ninth aspect, there is provided a compound, or selected combination of compounds, identifiable by the method of the eighth aspect.

According to a tenth aspect, there is provided a compound, or selected combination of compounds, identifiable by the method of the eighth aspect, said compound, or selected combination of compounds, selected from the group consisting of gamma-muurolene; cyperene; alpha-elemene; gamma-cadinene; delta-cadinene; alpha-gurjunene; alpha-guaiene; alpha-copaene; beta-cuabebene; caryophyllene; delta-guaiene; alpha-cedrene; 1,9,9-trimethyl-4,7-dimethano-2,3,5,6,7,8-hexahydroazulene; 1,1,5,5-tetramethyl-4-methano-2,3,4,6,7,10-hexahydronaphthalene; cuparene; beta-elemene; gamma-elemene; alpha-muurolene; beta-guaiene; 2,6-ditert-butyl-4-methyl phenol; 2,8-dimethyl-5-acetyl-bicylo[5,3,0] decadiene-1,8; methyl palmitate; ethyl palmitate; methyl heptadecadienoate; methyl octadecadienoate; ethyl octadecadienoate; a-ditertbutyl phthalate; dicapryl phthalate; diisocapryl phthalate; acetic acid; heptanoic acid; octanoic acid; nonanoic acid; palmitic acid; isoallyl-benzene; phenylethanone; octadecadienoic acid; 3-ene-nonanone-2; cyclododecanone; trans-nonenal-2; tridecene; 1-methyl-4-dioximethylthino-bicycle[2,2,2]octane; tetradecane; pentadecane; hexadecane; heptadecane; octadecane; nonadecane; eicosane; heneicosane; docosane; alpha, alpha-dimethyl-benzenemethanol; 1-methoxthyl-benzene; 2,2,2-tri-ethoxyl-ethanol; 1-methyl-4-isoallyl-cyclohexane[1,2]; ginsenoside Rb1; ginsenoside Rd; ginsenoside Re; ginsenoside Rg; ginsenoside Rg2; ginsenoside Rh1; gypenoside XV II; notoginsenoside R1; notoginsenoside R2; notoginsenoside R3; notoginsenoside R4; notoginsenoside R6; dencichine; beta-N-oxalo-L-alpha, beta-diaminopropionic acid; quercetin; beta-sitosterol; stigmasterol; daucostgerol; sanchinan A; dextrose aldehyde acide; picrates; choline; betaine; folic acid (2', 4'— Dihydroxy—5, 6—dimethoxyis of lavane); kumatakenin; tanshinone I; cryptotanshinone; hydroxytanshinone; methyltanshinonate; methylene tanshiquinone; przewatanshinquinone A, przewatanshinquinone B; miltirone; dihydrotanshinone I; tanshinol A; tanshinol B; tanshinol C; 3-α-hydroxy tanshinone II; nortanshinone; 1,2,15,16-tetrahydrotanshiquinone; tanshinone II A; tanshinone II B; cryptotanshinone; isotanshinone I; isotanshinone II; isocryptotanshinone; miltirone; isotanshinone; isocryptotanshinone; tanshiquinone A; tanshiquinone B; tanshiquinone C; saloilenone; danshenspiroketallactone; tanshilactone; salviol; tanshialdehyde; danshensuan A; danshensuan B; danshensuan C; protocatechuic acid; protocatechuic aldehyde; baicalin; β-sitosterol; ursolic acid; daucosterol; isoferulic acid; dihydroisotanshinone I; paeoniflorin; paeonol; paeonin; albiflorin; triterpenoids; sistosterol; oxypaeoniflorin; benzoylpaeoniflorin; benzoic acid; (β-sitosterol; gallotannin; pedunculagin; 1-O-galloylpedunculagin; eugeniin; tannin acid; resin; naphtha; cnidiumlactone; chuanxingol; 4-hyroxy-3-butylidene-phthalide; 4,5-dihydro-3-butylidene-phthalide; 3-butyl-phthalide; 7-hydroxy-3-butylidenephthalide; 3-butyl-4,5-dihydrophthalide; (Z)-4,5-dihydro-6,7-trans-dihydroxy-3-butylidene-phthalide; (Z)-4,5-dihydro-6,7-cis-dihydroxy-3-butylidene-phthalide; senkyunolide K; senkyunolide L; senkyunolide M; (Z)-6,7-epoxy-ligustilide; 3,6,7-trihydroxy-4,5,6,7-tetrahydro-3-butyl-phthalide; neocnidilide; 3-n-butyl-3-hydroxyl-4,5,6,7-tetrahydro-6,7-dihydroxyphthalide; (Z,Z')-diligustilide; (Z)-6,8',7,3'-diligustilide; wallichilide; (Z')-3,8-dihydro-6,6',7,3' a-diligustilide; chuanxingzine; tetramethyl-pyrazine; L-isoleucine-L-valine anhydride; adenine; L-valine-L-valine anhydride; trimethylamine; perlolyrine; ferulic acid; sedanonic acid; vanillic acid; caffeic acid; protocatechuic acid; linolenic acid; chrysophanol; methyl phenylacetate; sedanoic acid lactone; methyl pentadecanoate; ethyl p entadecanoate; ethyl heptadecanoate; ethyl isohepta-decanoate; ethyl octadecanoate; ethyl isoctadecanoate; methyl linolenate; lactone; asafetida acid; vanillin; bis-5,5'-formylfurperye ether; 5-hydroxymethyl-6-endo-3'-methoxy-4'-hydroxyphenyl-8-oxa-abicyclo(3,2,1)-oct-3-en-2-one; spathulenol; β-sitosterol; vitamin A; arasaponin A; paeonol; paeonoside; apiopaeonoside; paeconol; paeonolide (paeconol, arabian sugar and paeoniflorin); paeoniflorin; ozypaeoniflorin; benzoylpaeoniflorin; benzoyl-oxypaeoniflorin; gallic acid; 1,2,3,4,6-pentagalloylglucose; hirudin, heparin; rhynchophylline; isorhynchophylline; corynoxeine; isocorynoxeine; corynantheine; dihydrocorynantheine; hirsutine; hirsuteine; heter-Gouteng alkali; katsutoxin; secaline; lycine; cow sulfonic acid; cetylic acid; octadecanoic acid; cholesterin; lecithin; β-Asarone (cis-form); asaryl-aether; Caryophyllene; α-Humulene; Sekishone; 1-allyl-2,4,5-trimethyl-oxy-benzene; β-Asarone; asarone; cis-methylisoengenol; trans-methylisoeugenol; shyobunone; isoshyobuncne; epishyobunone; linalool; calamenene; β-gurjunene; δ-cadinene; calamendiol; isocalamendiol; preisocalamendiol; acoronene; acorone; acoragermacrone; acolamone; isoacolamone; caryophyllene; cis-metylisoeugenol; onjisaponin A, onjisaponin B, onjisaponin C, onjisaponin D, onjisaponin E, onjisaponin F, onjisaponin G; tenuifolin; 2-beta-2,7-dihydroxy-2,3-carboxyoleanolic acid 3-beta-O-glucoside; tenuigenin A; tenuigenin B; Saponin; onjixanthone; 1,6-dihydroxy-3,7-dimethoxy xanthone; 1-hydroxy-3, 6,7-trimethoxy xanthone; 5-anhydro-D-sorbitol; N-acetyl-D-glucosamine; 3,4,5-trimethoxy-cinnamic acid; polygalitol; tenuidine; bilirubin; biliverdin; cholic acid; chenodeoxycholic acid; lithocholic acid; stero-cholic acid; conjugated bile acid; cholestrol; mucigen; carotin; SMC-S; SMC-F; cow cholalic powder; cholalin; pig deoxygenated cholalin; cow sulfonic acid; cholesterin; Dalbergin; Nordalbergin; Isodalbergin; o-Methyldalbergin; Dalbergenone and Dalbergichromene, analogues of said selected compounds and any pharmaceutically acceptable salts thereof.

### GLOSSARY OF TERMS

This section is intended to provide guidance on the interpretation of the words and phrases set forth below (and where appropriate grammatical variants thereof).

As used herein, the term "about" as used in relation to a numerical value means, for example, ±50% or ±30% of the numerical value, preferably ±20%, more preferably ±10%, more preferably still ±5%, and most preferably +1%. Where necessary, the word "about" may be omitted from the definition of the invention.

The term "comprising" means "including" or "consisting". Thus, for example, a composition "comprising" X may consist exclusively of X or may include one or more additional components.

The term "treatment" includes any and all uses which remedy a disease state or symptoms, prevent the establishment of disease, or otherwise prevent, hinder, retard, or reverse the progression of disease or other undesirable symptoms in any way whatsoever. Hence, "treatment" includes prophylactic and therapeutic treatment.

By "a cerebral stroke patient" we include a patient who has suffered an ischemic or haemorrhagic cerebral stroke. A cerebral stroke is a sudden and permanent death of brain cells that occurs when the flow of blood is blocked and oxygen cannot be delivered to the brain. Ischaemic stroke most commonly occurs when the flow of blood is prevented by clotting (known as 'thrombosis' of the artery) or by a detached clot that lodges in an artery (referred to as an 'embolic stroke'). Haemorrhagic stroke results from rupture of an artery wall, and from blood leaking into the surrounding brain. Haemorrhagic stroke, like ischemic stroke, causes the death of tissue by depriving the brain of blood and oxygen, and results in a number of neurological disabilities (motor, speech) as well as functional disabilities.

The term "stroke" refers to the sudden death of tissue cells due to a lack of oxygen when the blood flow is impaired by blockage or rupture of an artery. Stroke is a vascular accident that can occur in the brain or in the cardiac system. The latter condition is medically known as "myocardial infarction" and more commonly known as a "heart attack". Because of the similarity of both stroke mechanisms, it may be useful to use NeuroAid^{®} to help patients with a heart stroke recovering better from their disability.

### DETAILED DESCRIPTION

The present invention provides a new combination treatment for patients having one or more of the following conditions: cerebral stroke, heart stroke, neurodegenerative diseases, brain trauma, nervous system trauma or conditions related to neuroplasticity.

The present invention provides methods and compositions for treating patients having a condition selected from the group consisting of cerebral stroke, heart stroke, neurodegenerative diseases, brain trauma, nervous system trauma and conditions related to neuroplasticity. The patients are administered with:
(i) a composition which comprises at least 4, 5, 6, 7, 8, 9, 10, 11, 12, 13 or 14 of the following ingredients: Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng); and
(ii) an agent used in Western medicine for the treatment of stroke.

This invention is based on the discovery that NeuroAid^{®} is not only efficacious in treating stroke patients but that it may be safely used in combination with treatments commonly used in Western medicine for treating stroke patients without adverse side effects such as haemostasis disorders or high blood pressure as described above.

As chronic forms of neurodegeneration such as Alzheimer's and Parkinson's disease (associated with cognitive dysfunction) share common mechanisms of neuronal death with acute forms of neurodegeneration which accompanies stroke, head trauma, cardiac arrest and subarachnoid hemorrhage, NeuroAid® may be useful to improve or treat these brain disorders as it is believed to have potential activity on neuroprotection or on plasticity. Neuroplasticity (also referred to as brain plasticity or cortical plasticity) refers to changes that occur in the organization of the brain and its circuits of neurons, in particular changes that occur to the location of specific information processing functions. This process supports the learning of new functions as the result of experience during development as mature animals and the creation of new information with healthy neurons by-passing damaged neurons affected by trauma or a medical condition.

Hence, in addition to treating cerebral stroke patients it is envisaged that NeuroAid^{®} or a NeuroAid^{®}-like composition (e.g. a composition according to (i) above), optionally in combination with available agents used in Western medicine, may be useful for the treatment of such neurodegenerative diseases. Typically, the Western medicine used in combination with NeuroAid^{®} or a NeuroAid^{®}-like composition is one that targets a different mechanism from NeuroAid^{®} or a NeuroAid^{®}-like composition. For example, the Western medicine may be antiplatelets and anticoagulants typically used in secondary stroke prevention, and neuroprotectants typically used in improving recovery potential in the acute phase of stroke via mechanisms described below.

### NeuroAid^{®} and similar compositions

The ingredients set forth in (i) above may be present in the composition in a relatively crude form (e.g. unprocessed or crushed herbs) or in a more refined form (e.g. purified extracts).

In one embodiment, NeuroAid^{®} is used. NeuroAid^{®} is a TCM product in capsule form comprising 9 herbal components and 5 animal components. NeuroAid^{®} comprises Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng).

NeuroAid^{®}, which may be registered under different names in different countries (e.g. in South Africa it is marketed as Strocaid^{®} or Danqi Piantan Jiaonang^{®}) is manufactured by and available commercially in the People's Republic of China from Tianjin Shitian Pharmaceutical Group Co., Ltd (located in the Jianxin Industrial area, Wangwenzhuang town, Xiqing district, Tianjin City, China; Postal Code 300381;). It is also available from Moleac Pte Ltd (formerly Molecular Acupuncture Pte Ltd), the main licensee outside of the People's Republic of China (11 Biopolis Way, Helios #09-08 Singapore 138667; Tel: 65 64789430; Fax: 6564789435).

For the avoidance of doubt, NeuroAid^{®} not only includes NeuroAid^{®} in the form in which it is currently marketed but also includes future formulations of NeuroAid^{®} which may, for example, be marketed by Tianjin Shitian Pharmaceutical Group Co., Ltd or Moleac Pte Ltd. Such future formulations may, for example, vary in dosage amounts or the concentration of its active ingredients etc.

### Combination therapy with NeuroAid^{®}

In the present invention, NeuroAid^{®} can be used in combination with one or more agents used in Western medicine for the treatment of stroke. By "an agent used in Western medicine", we include any form of mainstream medicine or Western medicine, including dietary supplements. For the avoidance of doubt, by "an agent used in Western medicine", we do not include medicaments used in TCM medicaments or the like.

Examples of suitable agents include antiplatelets, anticoagulants and neuroprotectants. One, two, three or more of such further agents may, for instance, be used in combination with NeuroAid^{®}. Thus, the following are envisaged as suitable examples for use as combination partner (ii): an antiplatelet; an anticoagulant; a neuroprotectant; an antiplatelet in combination with an anticoagulant; an antiplatelet in combination with a neuroprotectant; an anticoagulant in combination with a neuroprotectant; and, an antiplatelet in combination with an anticoagulant and a neuroprotectant.

### Antiplatelet agents

Antiplatelet agents are medications that block the formation of blood clots by preventing the clumping of platelets. Examples of antiplatelet agents include without limitations: Aspirin, the thienopyridine derivatives such as ticlopodine (Tidid) and dopidogrel (Plavix), the Phosphodiesterase III inhibitors such as Cilostazol (Pletal), Adenosine re-uptake inhibitors such as Dipyridamole (Persantine or Aggrenox (in combination with aspirin)), and the glycoprotein IIb/IIIa inhibitors such as Abciximab (ReoPro), Eptifibatide (Integrilin) and Tirofiban (Aggrastat) and orally active RGD mimetic prodrugs such as Orbofiban, Sibrafiban, SR121566, or Roxifiban.

These agents differ in the way in which they prevent platelets from clumping: for example, Aspirin (Cyclo-oxygenase inhibitor) blocks thromboxan A-2 by inhibiting the enzyme cyclo-oxygenase-1 (COX-1), the thienopyridine derivatives (ADP inhibitors) block the adenosine diphosphate (ADP) receptor on the surface of platelets membrane, and glycoprotein IIB/IIa inhibitors prevent platelet aggregation by inhibiting a different receptor at the surface of platelets (the attachment of glycoprotein IIb/IIIa to its receptor is the final step in all pathways that cause platelets aggregation).

One or more antiplatelet agents can be employed in the present invention, for instance, a combination of, for example, 2 or 3 or more antiplatelet agents may be employed.

### Anticoagulant therapies

Antiplatelet agents are part of anticoagulation therapies. There are two other groups of anticoagulant agents which may also be employed in the present invention:
● Inhibitors of clotting factor synthesis such as without limitation, vitamin K antagonists like coumarins or indanedione derivatives (Warfarin or Coumadin).
● Inhibitors of thrombin that include several products such as, but not limited to Heparin (Standard Unfractionated Heparin (UFH), Low Molecular Weight Heparin (LMWH) such as Enoxaparin, Tinzaparin) or to recombinant forms of hirudin such as desirudin and lepirudin (Refludan).

### Neuroprotectants

Using various mechanisms, neuroprotectants are compounds that preserve neuronal tissue at risk of dying during stroke and in the aftermath of stroke. Some neuroprotectant agents are sometimes used to treat human stroke patients and include antioxidants (e.g. selenium, 30 vitamin E, vitamin C, glutathione, cysteine, flavinoids, quinolines, enzymes with; reducing activity, etc), N-methyl-D-aspartate Receptor Antagonists (Dextrorphan, Selfotel, Magnesium), Narcotic Receptor antagonist (Nalmefene (Cervene), Ca-channel blockers, Na-channel modulators (Lubeluzole), Alpha-aminobutyric acid agonist (Clomethiazole), glutamate receptor modulators, serotonin receptor agonists (repinotan), phospholipids, free-radical scavenger (Tirilazad, and NXY-059), astrocyte activation inhobitor (ONO 2506), monoclonal antibodies such as anti-ICAM-1 (Enlimomab), Human anti-leukocytic antibody, Hu23F2G, membrane stabilization agent CDP-choline (Citicholine), Fibroblast growth factor (Fiblast), unsaturated- and polyunsaturated fatty acids, estrogens and selective estrogen receptor modulators (SEAMS), progestins, thyroid hormone and thyroid hormone- mimicking compounds, cyclosporin A and derivatives, thalidomide and derivatives, methylxanthines, Mono-Amine-Oxydase inhibitors (IMAO), serotonin-, noradrenaline and dopamine uptake blockers, dopamine I agonists, L-DOPA, nicotine and derivatives, and NO synthase modulators.

### Modes of Administration

The combination partners (i) and (ii) may be present in a single formulation or may be present as separate formulations. In one embodiment there may be a synergistic effect. As mentioned above, combination partner (ii) may comprise more than one agent, for example, two antiplatelet agents, or an antiplatelet agent and a neuroprotectant may be used.

The combination partners (i) and (ii) may be administered to the patient at the same time (e.g. simultaneously) or at different times (e.g. sequentially) and over different periods of time, which may be separate from one another or overlapping. The combination partners (i) and (ii) may be administered in any order.

The combination partner (ii) utilized and the appropriate administration route and dose level will be known to those in the art or could be readily determined by one skilled in the art. Typically, as is well known in the medical art, dosage regimens may depend on various factors including the patient's size, body surface area, age, the particular compound to be administered, sex, time and route of administration, general health, and other drugs being administered concurrently. While individual needs vary, determination of optimal ranges of effective amounts of each component is within the skill of the art. The dosage would be similar to that administered when the agent is used without NeuroAid^{®}.

Dosage amounts for ticlopidine and for dipyridamole are described in the Physicians' Desk Reference, as are dosage amounts for other antiplatelet and neuroprotectant agents. Dosage amounts of aspirin for the indicated effects are known to those skilled in the medical arts, and generally range from about 20mg to about 325 mg per day. For example, a formulation may contain about 20mg, 30mg, 80 mg, 160 mg, 250 mg, 300 mg, 325 mg or 350 mg of aspirin.

NeuroAid^{®} may be administered orally as such, typically with four 0.4g capsules being taken 3 times a day. For patients with swallowing difficulties, capsules may be opened and powder diluted in water that can be drunk as such or injected via a gastric tube. Hence, a daily dose of about 4.8g is envisaged. In one embodiment, the patient's daily dose of NeuroAid^{®} (or other composition according to (i) above) is about 2g to 8g; 3g to 7g; 4g to 6g; 4.25g to 5.75g; 4.5g to 5.25g; 4.5g to 5g; 4.6g to 4.10g; or 4.7g to 4.9g. A "daily dose" can be a single tablet or capsule etc. or multiple tablets or capsules etc. to be taken on a given day. Suitably, the composition according to (i) is taken orally.

In one embodiment, each course of NeuroAid^{®} treatment lasts about 4 weeks. Typically 3 courses are administered, most commonly back to back. No therapeutic window is required but additional courses can be added even after a few days of treatment cessation. Hence, in one embodiment, each NeuroAid^{®} treatment lasts about 12 weeks. In another embodiment, the treatment course of NeuroAid^{®} (or other composition according to (i) above) is about 4 to 24 weeks; 7 to 16 weeks; 9 to 15 weeks; 10 to 14 weeks; or 11 to 13 weeks.

In instances of ischemic stroke, treatment with anti-platelet drugs usually commences as soon as possible after onset of stroke symptoms while in instances of haemorrhagic stroke, anticoagulation treatments such as Coumadin or Heparin, are highly contra-indicated and discontinued immediately if they were part of patient's usual treatment. Protamine and vitamin K may be given to reduce bleeding in patients with anticoagulant-induced bleeding.

In addition to combination partners (i) and (ii), other compounds may be administered, for example, proton pump inhibitors such as Nexium, Protonix and Aciphex. Daily doses of proton pump inhibitors are typically administered to reduce the risk of ulcer development and bleeding in patients under long-term low-dose aspirin or antiplatelet therapy.

### BRIEF DESCRIPTION OF DRAWINGS

* Figure 1 Phase II clinical trial results on use of NeuroAid^{®} versus BNJ (a) Neurological deficit improvement (DTER scores) before and after treatment with NeuroAid^{®} or BNJ (b) Functional outcomes before and after treatment with NeuroAid^{®} or BNJ.
* Figure 2 Phase III clinical trial results on use of NeuroAid^{®} versus BNJ (a) Neurological deficit improvement (DTER scores) before and after treatment with NeuroAid^{®} or BNJ (b) Functional outcomes before and after treatment with NeuroAid^{®} or BNJ.
Figure 3 Clinical study results on the interaction between NeuroAid^{®} and aspirin in healthy volunteers and its effect on blood pressure. (a) systolic blood pressure (b) diastolic blood pressure.

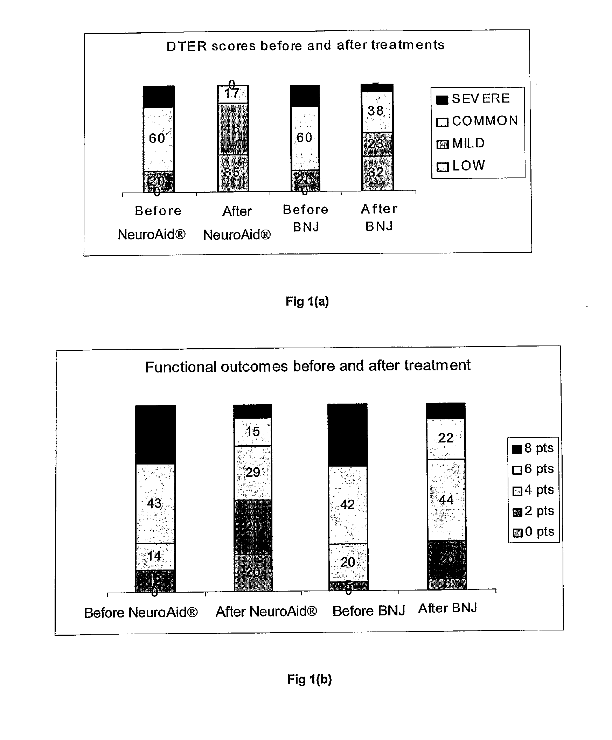

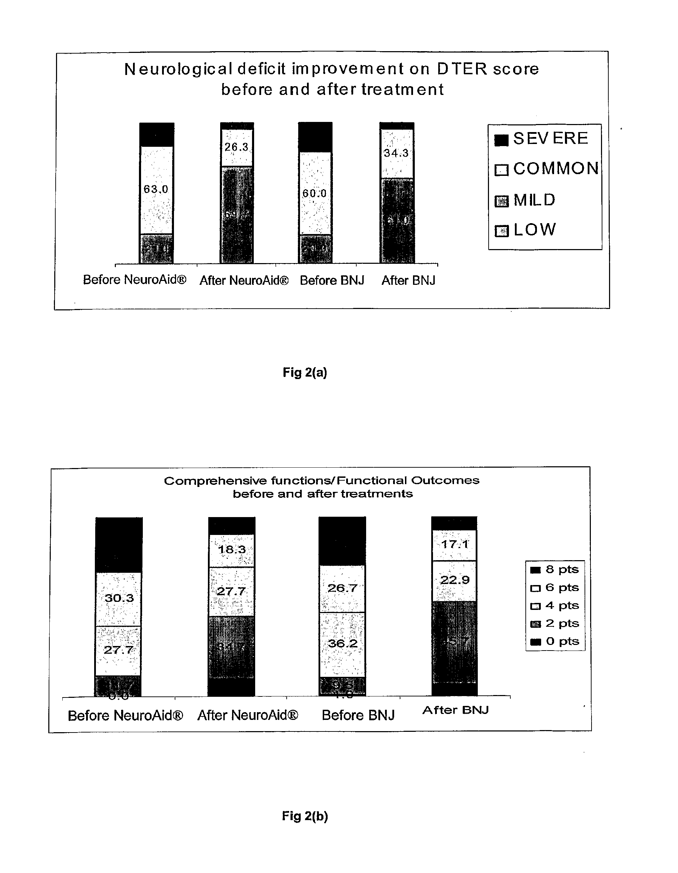

### * For Figures 1 and 2:

Level of severity of stroke (DTER scores) = LOW, MILD, COMMON, SEVERE Functional outcomes: 0 pts = able to take care of oneself and speak freely; 2 pts = able to live independently and to do some simple work with some incomplete functions; 4 pts = able to walk and take care of oneself but must be helped partially; 6 pts = able to stand and take a step but must be taken care of at all times; 8 pts = confined to bed.

### EXAMPLES

In the following examples, tables and drawings, the term "NeuroAid^{®}" refers to a composition that is the same as NeuroAid^{®}.

### EXAMPLE 1

### Phase II trial: NeuroAid^{®} versus Buchang Naoxintong Jioanang (BNJ)

A randomized, double-blinded, stratified, control design was adopted for the clinical trial on the efficacy of NeuroAid^{®} in treating patients suffering from apoplexy compared to BNJ which is known for its effectiveness in treating patients suffering from apoplexy (see Example 3). A total of 200 subjects were involved; 100 cases were treated with NeuroAid^{®} while 100 cases were treated with BNJ (control). BNJ is produced and provided by Xianyang Buchang Pharmaceutical Co., Ltd. Four capsules of each drug were administered 3 times daily, with each course of treatment lasting 4 weeks.

The evaluation criteria for neurological and functional recovery from apoplexy (DTER scoring diagnostic standard) and TCM symptom therapeutic effects (TCM diagnostic symptom scoring standard) were assessed in accordance with the Clinical Guiding Principles for the Treatment of Apoplexy with New Chinese Herbs promulgated by the Ministry of Health of the PRC in 1993. Severity of symptoms in the DTER scoring standard was classified according to 4 levels (SEVERE, COMMON, MILD and LOW) while functional outcomes were classified in points from 0 to 8.

The data (Figure 1) demonstrated NeuroAid^{®}'s clinical efficacy and superiority in improving patients' neurological deficit and functional (autonomy/dependency post-stroke) outcomes versus that of the control treatment BNJ.

### EXAMPLE 2

### Phase III trial: NeuroAid^{®} versus BNJ

A randomized, double-blinded, stratified, control design was adopted. A total of 405 subjects were involved, where 300 cases were treated NeuroAid^{®} while 105 cases were treated with the control drug BNJ produced and provided by Xianyang Buchang Pharmaceutical Co., Ltd. Four capsules of each drug were administered 3 times daily, with each course of treatment lasting 4 weeks.

The evaluation criteria for neurological and functional recovery from apoplexy (DTER scoring diagnostic standard) and TCM symptom therapeutic effects (TCM diagnostic symptom scoring standard) were assessed as in the Phase II trial.

The data (Figure 2) demonstrated that NeuroAid^{®} was superior to BNJ in improving the patients' neurological deficit particularly in helping patients recover from their hemi-paralysis. With regard to functional outcomes, even if NeuroAid^{®} failed to demonstrate any superiority to BNJ, both treatments had comparable effect and about 50% of the stroke patients included in the study returned to a functionality dependent state after 4 weeks of treatment (comprehensive function score similar to Rankin score used in the West equal to 0 or 2).

No adverse effects of treatment with NeuroAid^{®} were observed in the Phase II or Phase III trials. The finding of a broad therapeutic window (>14 days after stroke onset) in both trials also gives a huge advantage over fibrinolytic agents, which have to be given within the first 3 to 6 hours.

### EXAMPLE 3

### Comparative study on efficacy of BNJ versus Citicoline and aspirin

A randomized, double-blinded, stratified, control design was adopted for the comparative study on the efficacy of BNJ and Citicoline in treating patients with apoplexy. Citicoline is the only putative neuro-protectant that has shown results in Western randomized, double-blinded trials given within 24 hours after symptom onset. Davalos et al. (Oral Citicoline in Acute Ischemic Stroke. An Individual Patient Data Pooling Analysis of Clinical Trials (2002) *Stroke* 33:2850) documents the ability of Citicoline to improve complete recovery at 3 months.

A first comparative study performed in the PRC involved 150 subjects treated with Citicoline (0.5g IV) for 15 days in combination with a TCM (Xueshuantong) and 160 subjects treated with Citicoline (0.5g IV) for 15 days in combination with Xueshuantong and BNJ. The latter group treated with BNJ showed improvements in scores on a neuro-functional defects scale, plasma viscosity level and cholesterol level.

A second comparative study compared 30 subjects treated with aspirin (150mg), Citicoline (0.75g IV) and salvia miltiorrhizae injection (60mL), with 30 subjects treated with aspirin (150mg), Citicoline (0.75g IV), salvia miltiorrhizae injection (60mL) and BNJ for 1 month. The BNJ-treated group showed significantly better results in neurological and functional outcomes.

Citicoline (CDP-choline) is a key intermediary in the biosynthesis of phosphatidylcholine, an important component of the neural cell membrane that stabilizes cells membranes and inhibits the formation of cytotoxic free fatty acids. It has been shown to produce beneficial effects in both animal models and clinical stroke trials. A significant difference between the groups, favoring citicoline treatment, was seen in terms of functional outcome as measured by the Barthel Index and Rankin scale, neurologic evaluation as measured by the National Institutes of Health (NIH) stroke scale, and cognitive function as measured by the Mini Mental Status Examination. ¹As efficacy trials on Citicoline have been demonstrated to be superior to the placebo, it can be inferred from the above two comparative studies that treatment with BNJ would likewise be superior to a placebo.

### EXAMPLE 4

### Safety Study: Phase IV open-label study on effect of NeuroAid^{®} on hemostasis

Blood samples of 30 healthy individuals were collected before they received NeuroAid^{®} and 2 and 8 hours after NeuroAid^{®} initiation (4 capsules). Five coagulation tests were performed on the blood samples.

**Table 1.**

| **Quick Prothrombine Time** | | | |
|---|---|---|---|
| *Blood Sampling* | *First (%)* | *Second (%)* | *Third* (%) |
| Average | 12.75 | 12.71 | 12.92 |
| Std Deviation | 0.68 | 0.72 | 0.67 |

| **Activated Partial Prothrombine Time** | | | |
|---|---|---|---|
| *Blood Sampling* | *First (%)* | *Second (%)* | *Third (%)* |
| Average | 37.64 | 37.15 | 38.66 |
| Std Deviation | 4.28 | 4.44 | 4.26 |

| **Fibrinogen** | | | |
|---|---|---|---|
| *Blood Sampling* | *First* (%) | *Second (%)* | *Third (%)* |
| Average | 2.98 | 3.05 | 3.06 |
| Std Deviation | 0.61 | 0.59 | 0.57 |

| **Platelet Aggregation** | | | |
|---|---|---|---|
| *Blood Sampling* | *First (%)* | *Second (%)* | *Third* (%) |
| Average | 63.01 | 62.19 | 61.52 |
| Std Deviation | 15.50 | 12.08 | 13.37 |

| **D-Dimer** | | | |
|---|---|---|---|
| *Blood Sampling* | *First* (%) | *Second (%)* | *Third* (%) |
| Control Device | 2.7 | 2.4 | 1.9 |

The results confirmed that NeuroAid^{®} has no effect on hemostasis blood factors and thus does not increase bleeding risks.

### EXAMPLE 5

### Safety Study: Phase IV open-label study on potential interaction between NeuroAid^{®} and acetylsalicylic acid (aspirin) in healthy volunteers and its effect on hemostasis and blood pressure

An open randomized one-day study was conducted on 11 healthy volunteers receiving NeuroAid^{®} treatment (12 capsules per day) and aspirin (ASA 300mg per day) from day 1 to day 5, and 11 healthy volunteers receiving aspirin alone (300mg per day) from day 1 to day 5. Blood samples of the subjects were collected before they received treatment and 2 and 8 hours after treatment initiation. The blood samples were analyzed with the 5 coagulation tests, namely quick prothrombine time, activated partial prothrombine time, fibrinogen dosage, platelet aggregation and D-dimer, to identify the coagulation mechanisms triggered by potential interaction between NeuroAid^{®} and aspirin. Blood pressure of the 22 subjects was also measured before treatment, and 2, 4, 6 and 8 hours after treatment initiation.

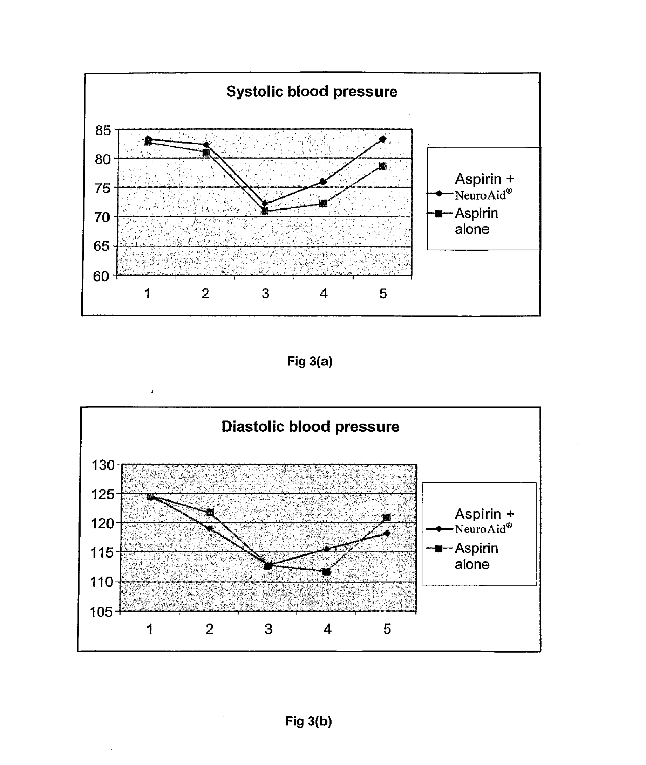

Results of this trial show that NeuroAid^{®} can be safely associated with aspirin and does not have any interaction with aspirin regarding blood coagulation (Table 2) and blood pressure (Figure 3).

**Table 2.**

| **Quick Prothrombine Time (Seconds)** | |
|---|---|
| Aspirin alone | Mean |
| T = 0 | 12.9 |
| T + 2 hours | 12.6 |
| T + 8 hours | 12.4 |
| Aspirin + NeuroAid^{®} | Mean |
| T = 0 | 12.7 |
| T + 2 hours | 12.6 |
| T + 8 hours | 12.4 |

| **Activated Partial Prothrombine Time (Seconds)** | |
|---|---|
| Aspirin alone | Mean |
| T = 0 | 38.8 |
| T + 2 hours | 39.0 |
| T + 8 hours | 38.2 |
| Aspirin + NeuroAid^{®} | Mean |
| T = 0 | 37.1 |
| T + 2 hours | 38.2 |
| T + 8 hours | 37.2 |

| **Fibrinogen (G/L)** | |
|---|---|
| Aspirin alone | Mean |
| T=0 | 3.2 |
| T + 2 hours | 3.2 |
| T + 8 hours | 3.0 |
| Aspirin + NeuroAic^{®} | Mean |
| T=0 | 3.2 |
| T + 2 hours | 3.0 |
| T + 8 hours | 2.9 |

| **Platelet Aggregation (Percentage)** | |
|---|---|
| Aspirin alone | Mean |
| T = 0 | 62.82 |
| T + 2 hours | 62.87 |
| T + 8 hours | 48.01 |
| Aspirin + NeuroAid^{®} | Mean |
| T = 0 | 61.82 |
| T + 2 hours | 58.28 |
| T + 8 hours | 46.98 |

| **D-Dimer (µG/L)** | |
|---|---|
| Aspirin alone | Mean |
| T=0 | 0.16 |
| T + 2 hours | 0.16 |
| T + 8 hours | 0.16 |
| Aspirin + NeuroAid^{®} | Mean |
| T=0 | 0.13 |
| T + 2 hours | 0.09 |
| T + 8 hours | 0.15 |

### EXAMPLE 6

### Safety Study: Pilot open-label study on potential interaction between NeuroAid^{®} and Western drugs in ischemic stroke patients and its effect on hemostasis and blood pressure

A pilot open-label study was conducted on 10 ischemic stroke patients within the first week of the onset of stroke (early phase). The test patients were selected based on the following criteria:
a) aged above 18 years old;
b) have had a cerebral infarction with compatible imaging on Computed Tomography (CT) scan or Magnetic Resonance Imaging (MRI); and
c) the time window between stroke onset and the open-label study was less than one week,

Conversely, patients that were excluded from the study include:
a) female patients who were pregnant, lactating or nursing;
b) patients showing signs of intra-cerebral hemorrhage on brain Computed Tomography (CT) scan or Magnetic Resonance Imaging (MRI);
c) patients having a history of easy bruising or blood coagulation disorders;
d) patients receiving other Traditional Chinese Medicine than NeuroAid^{®};
e) patients who received thrombolysis;
f) patients who have used NeuroAid^{®} within a 3-month period prior to screening and enrolment in the open-label study.

Each patient received 4 capsules of NeuroAid^{®} 3 times a day for one month in addition to the other Western medicine the patient was receiving. The Western medicine includes platelet aggregation inhibitors, nitrates, oral anti-hypertensive drugs, lipid regulating drugs, oral anti-diabetic, or anti-convulsant drugs.

Safety assessment tests were performed on blood samples collected from the test patients at three intervals: a) before NeuroAid^{®} intake (to form the baseline); b) 1 week after NeuroAid^{®} initiation; and c) 4 weeks after NeuroAid^{®} initiation. The blood samples were analyzed using the following hematology and biochemical laboratory analytical tests:
a) Prothrombine Time (PT);
b) Activated Partial Prothrombine Time (APPT);
c) fibrinogene dosage;
d) platelet aggregation;
e) D-dimer test;
f) blood cell count;
g) creatinine;
h) SGOT SGPT;
i) glycemia; and
j) CRP-C-reactive protein.

The results of the open-label study (Table 3) demonstrated that NeuroAid^{®} does not have any effect on renal and hepatic functions, glycemia, and C-Reactive-protein when used in combination with Western medicine for managing risk factors of secondary stroke. For example, in the five coagulation tests (Prothrombine Time (PT), Activated Partial Prothrombine Time (APPT), fibrinogene dosage, platelet aggregation, D-dimer test) performed on Patients 4 and 5 (both receiving NeuroAid^{®} and four different types of Western medicine) (Table 3, columns 5 and 6), the test readings taken 1 week and 4 weeks after NeuroAid^{®} initiation do not differ significantly from the baseline readings taken before the start of NeuroAid^{®} intake.

The statistical analysis of the readings for the open-label study (Table 4) also show that the readings from all 10 patients do not differ significantly from the mean values. For example, in the Prothrombine Time (PT) test, the Standard Deviation (SD) values after 1 week and 4 weeks of NeuroAid^{®} initiation are 1.09 and 0.77, respectively, which do not deviate significantly from the baseline value of 0.88. Similarly, in the fibrinogen dosage test, the Standard Deviation (SD) values after 1 week and 4 weeks of NeuroAid^{®} initiation are 1.22 and 1.11, respectively, which do not deviate significantly from the baseline value of 1.13.

Hence, the results of the open-label study further demonstrated that NeuroAid^{®} can be safely used in combination with Western medicine such as platelet aggregation inhibitors, nitrates, oral anti-hypertensive drugs, lipid regulating drugs, oral anti-diabetic, or anti-convulsant drugs.

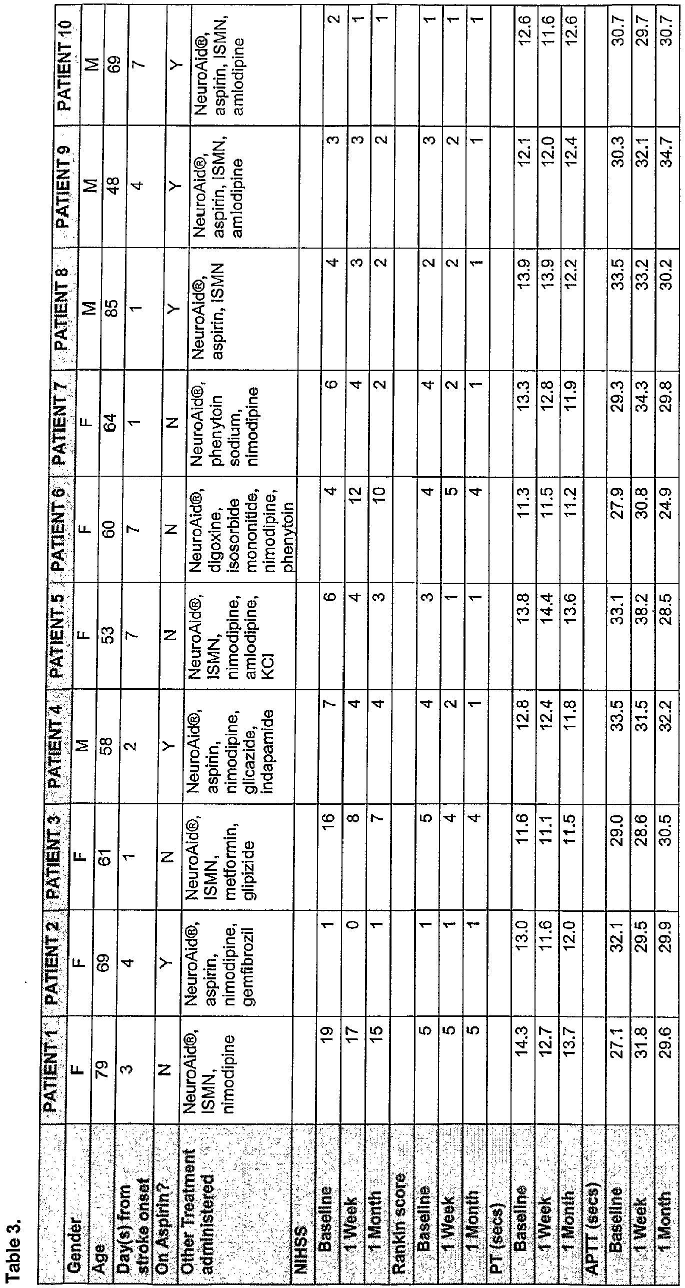

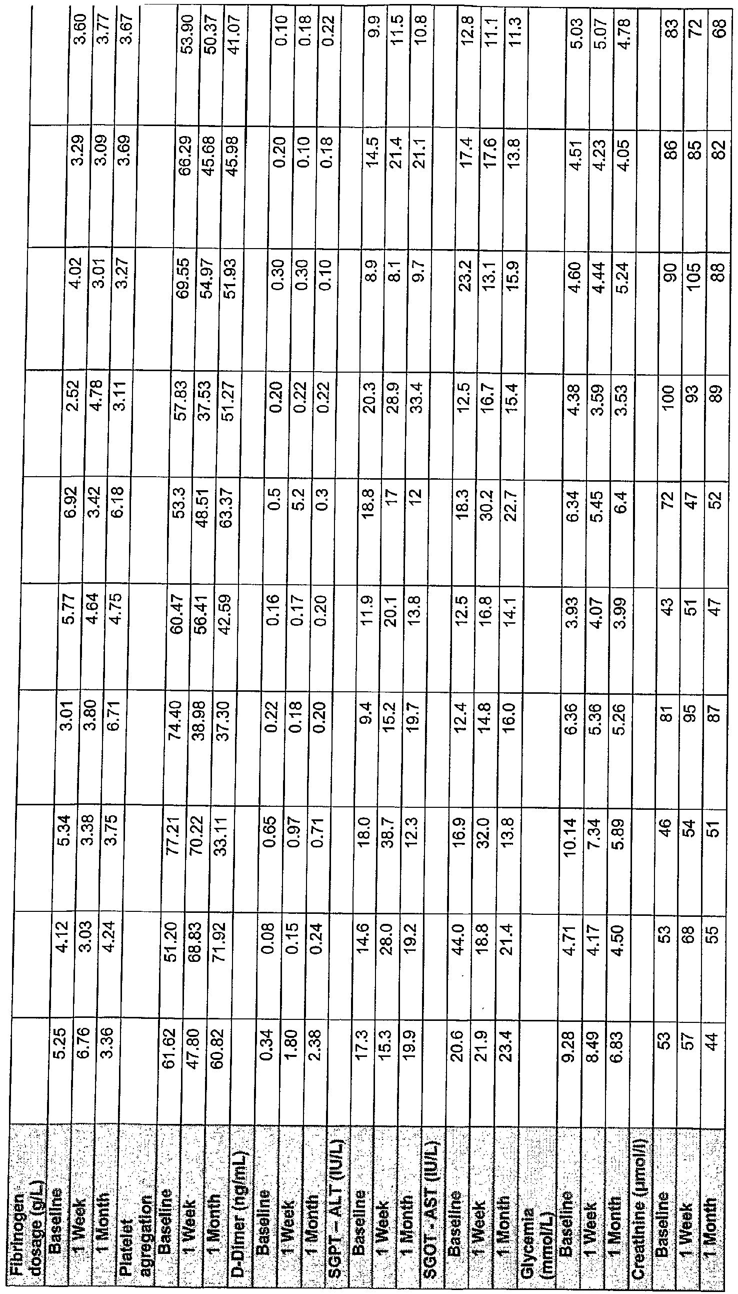

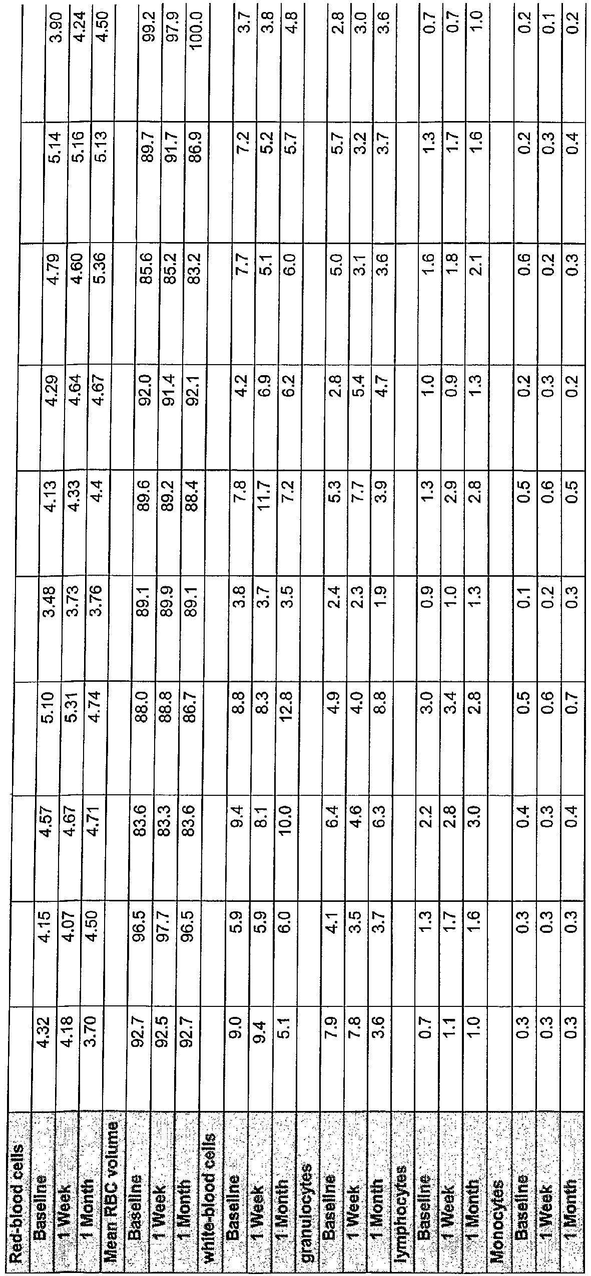

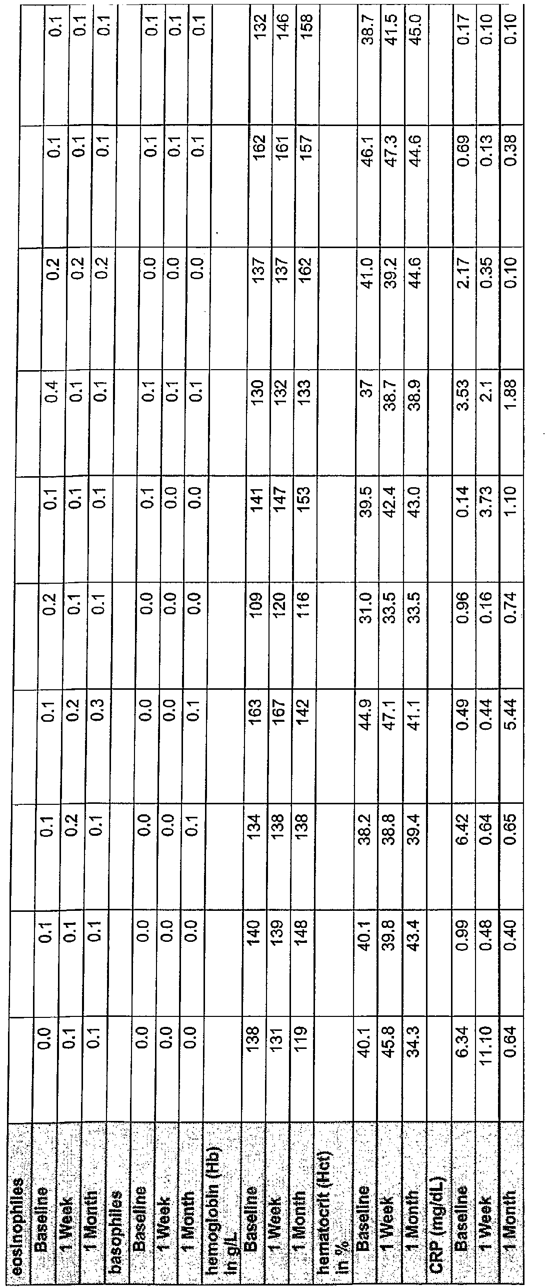

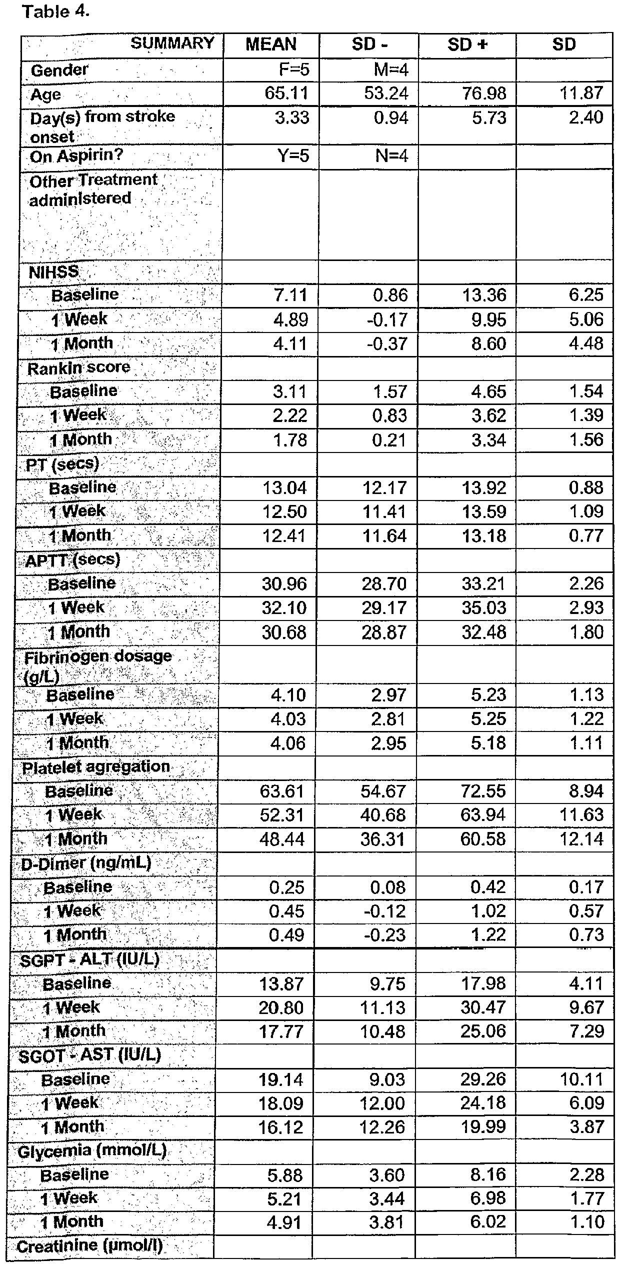

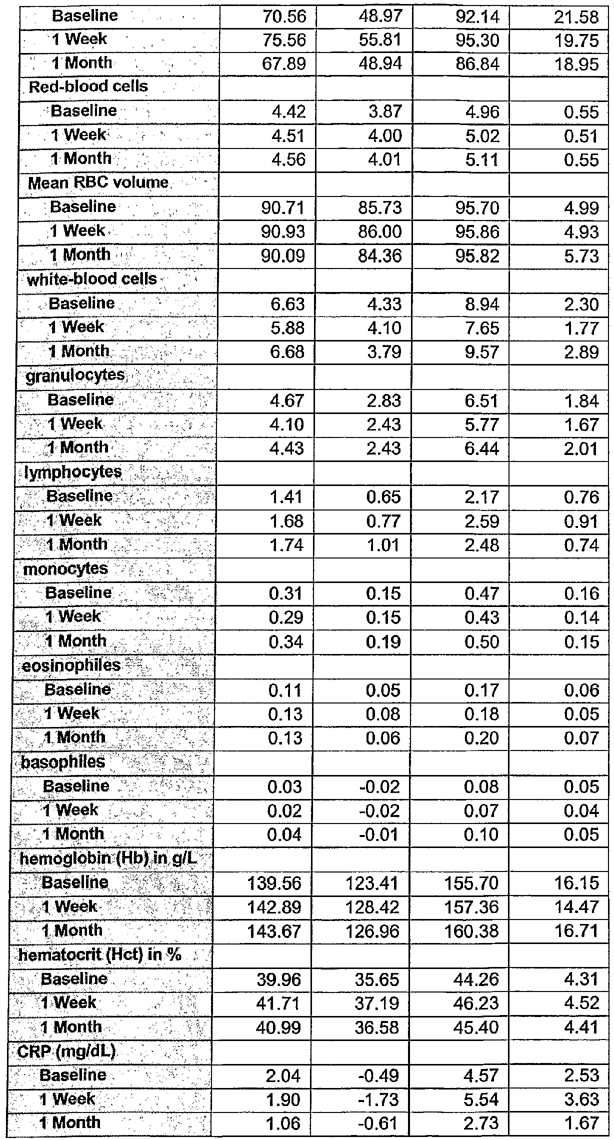

It will be understood that the invention has been described by way of example only and modifications may be made whilst remaining within the scope and spirit of the invention.

The present application and invention further includes the subject matter of the following numbered clauses:
1. A method of treating a patient having a condition selected from the group of cerebral stroke, heart stroke, neurodegenerative diseases, brain trauma, nervous system trauma and conditions related to neuroplasticity, the method comprising administering to the patient, (i) a composition which comprises at least 4, 5, 6, 7, 8, 9, 10, 11, 12, 13 or 14 of the following ingredients: Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng); and (ii) an agent used in Western medicine for the treatment of cerebral stroke, heart stroke, neurodegenerative diseases, brain trauma, nervous system trauma or conditions related to neuroplasticity.
2. Use of: (i) a composition which comprises at least 4, 5, 6, 7, 8, 9, 10, 11, 12, 13 or 14 of the following ingredients: Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng); and (ii) an agent used in Western medicine for the treatment of stroke, in the manufacture of a medicament for use in treating patients with cerebral stroke, heart stroke, neurodegenerative diseases, brain trauma, nervous system trauma or conditions related to neuroplasticity.
3. Use of: a composition which comprises at least 4, 5, 6, 7, 8, 9, 10, 11, 12, 13 or 14 of the following ingredients: Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng), in the manufacture of a medicament for treating a patient having a condition selected from the group consisting of cerebral stroke, heart stroke, neurodegenerative diseases, brain trauma, nervous system trauma and conditions related to neuroplasticity, where the patient also receives an agent used in Western medicine for the treatment of patients with cerebral stroke, heart stroke, neurodegenerative diseases, brain trauma, nervous system trauma or conditions related to neuroplasticity.
4. Use of an agent used in Western medicine for the treatment of cerebral stroke, heart stroke, neurodegenerative diseases, brain trauma, nervous system trauma or conditions related to neuroplasticity in the manufacture of a medicament for treating a a patient having a condition selected from the group consisting of cerebral stroke, heart stroke, neurodegenerative diseases, brain trauma, nervous system trauma and conditions related to neuroplasticity where the patient also receives a composition which comprises at least 4, 5, 6, 7, 8, 9, 10, 11, 12, 13 or 14 of the following ingredients: Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng).
5. A product comprising as a combined preparation: (i) a composition which comprises at least 4, 5, 6, 7, 8, 9, 10, 11, 12, 13 or 14 of the following ingredients: Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng); and (ii) an agent used in Western medicine for the treatment of stroke, for simultaneous, separate or sequential use in the treatment of patient with a cerebral stroke, heart stroke, neurodegenerative diseases, brain trauma, nervous system trauma or conditions related to neuroplasticity, and optionally instructions for use.
6. A method of treating a patient having a condition selected from the group of cerebral stroke, heart stroke, neurodegenerative diseases, brain trauma, nervous system trauma and conditions related to neuroplasticity, the method comprising administering to the patient, (i) NeuroAid^{®}; and (ii) an agent used in Western medicine for the treatment of cerebral stroke, heart stroke, neurodegenerative diseases, brain trauma, nervous system trauma or conditions related to neuroplasticity.
7. A product comprising as a combined preparation (i) NeuroAid^{®}; and (ii) an agent used in Western medicine for the treatment of stroke, for simultaneous, separate or sequential use as a medicament for the treatment of a patient having a condition selected from the group of cerebral stroke, heart stroke, neurodegenerative diseases, brain trauma, nervous system trauma and conditions related to neuroplasticity, and optionally instructions for use.
8. A product according to any one of clauses 5 or 7 wherein (i) and (ii) are present as separate formulations.
9. Use of any one of clauses 2, 3 or 4 wherein the medicament comprises (i) and (ii) as separate formulations suitable for simultaneous, separate or sequential administration.
10. A method of identifying at least one compound for treating at least one of cerebral stroke, heart stroke, neurodegenerative diseases, brain trauma, nervous system trauma or conditions related to neuroplasticity, the method comprising the step of selecting one or more isolated compounds from one or more herbs selected from the group consisting of Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong), Radix et Rhizoma Notoginseng (Sanqi), Cortex moutan (Peony or Mudanpi), Wood of Odoriferous Rosewood (Jiang Xiang), dried body of Scorpion (Quan Xie), Radix Polygalae root (Yuan Zhi), Grassleaf sweetflag rhizome (Shi Changpu), Leeches (Hirudo or Shuizhi), Ground Beetle (or Tu Bie Chong), Natural or Artificial Cow-bezoar (calculus Bovis artifactus or Rengong Niuhuang), Gambirplant stem with hooks (Ramulus uncariae cum uncis or Gou Teng).
11. A compound, or selected combination of compounds, identifiable by the method of clause 10.
12. A compound, or selected combination of compounds, identifiable by the method of clause 10, said compound, or selected combination of compounds, selected from the group consisting of gamma-muurolene; cyperene; alpha-elemene; gamma-cadinene; delta-cadinene; alpha-gurjunene; alpha-guaiene; alpha-copaene; beta-cuabebene; caryophyllene; delta-guaiene; alpha-cedrene; 1,9,9-trimethyl-4,7-dimethano-2,3,5,6,7,8-hexahydroazulene; 1,1,5,5-tetramethyl-4-methano-2,3,4,6,7,10-hexahydronaphthalene; cuparene; beta-elemene; gamma-elemene; alpha-muurolene; beta-guaiene; 2,6-ditert-butyl-4-methyl phenol; 2,8-dimethyl-5-acetyl-bicylo[5,3,0] decadiene-1,8; methyl palmitate; ethyl palmitate; methyl heptadecadienoate; methyl octadecadienoate; ethyl octadecadienoate; a-ditertbutyl phthalate; dicapryl phthalate; diisocapryl phthalate; acetic acid; heptanoic acid; octanoic acid; nonanoic acid; palmitic acid; isoallyl-benzene; phenylethanone; octadecadienoic acid; 3-ene-nonanone-2; cyclododecanone; trans-nonenal-2; tridecene; 1-methyl-4-dioximethylthino-bicycle[2,2,2]octane; tetradecane; pentadecane; hexadecane; heptadecane; octadecane; nonadecane; eicosane; heneicosane; docosane; alpha, alpha-dimethyl-benzenemethanol; 1-methoxthyl-benzene; 2,2,2-tri-ethoxyl-ethanol; 1-methyl-4-isoallyl-cyclohexane[1,2]; ginsenoside Rb1; ginsenoside Rd; ginsenoside Re; ginsenoside Rg; ginsenoside Rg2; ginsenoside Rh1; gypenoside XV II; notoginsenoside R1; notoginsenoside R2; notoginsenoside R3; notoginsenoside R4; notoginsenoside R6; dencichine; beta-N-oxalo-L-alpha, beta-diaminopropionic acid; quercetin; beta-sitosterol; stigmasterol; daucostgerol; sanchinan A; dextrose aldehyde acide; picrates; choline; betaine; folic acid (2', 4'— Dihydroxy—5, 6—dimethoxyis of lavane); kumatakenin; tanshinone I; cryptotanshinone; hydroxytanshinone; methyltanshinonate; methylene tanshiquinone; przewatanshinquinone A, przewatanshinquinone B; miltirone; dihydrotanshinone I; tanshinol A; tanshinol B; tanshinol C; 3-α-hydroxy tanshinone II; nortanshinone; 1,2,15,16-tetrahydrotanshiquinone; tanshinone II A; tanshinone II B; cryptotanshinone; isotanshinone I; isotanshinone II; isocryptotanshinone; miltirone; isotanshinone; isocryptotanshinone; tanshiquinone A; tanshiquinone B; tanshiquinone C; saloilenone; danshenspiroketallactone; tanshilactone; salviol; tanshialdehyde; danshensuan A; danshensuan B; danshensuan C; protocatechuic acid; protocatechuic aldehyde; baicalin; β-sitosterol; ursolic acid; daucosterol; isoferulic acid; dihydroisotanshinone I; paeoniflorin; paeonol; paeonin; albiflorin; triterpenoids; sistosterol; oxypaeoniflorin; benzoylpaeoniflorin; benzoic acid; β-sitosterol; gallotannin; pedunculagin; 1-O-galloylpedunculagin; eugeniin; tannin acid; resin; naphtha; cnidiumlactone; chuanxingol; 4-hyroxy-3-butylidene-phthalide; 4,5-dihydro-3-butylidene-phthalide; 3-butyl-phthalide; 7-hydroxy-3-butylidenephthalide; 3-butyl-4,5-dihydrophthalide; (Z)-4,5-dihydro-6,7-trans-dihydroxy-3-butylidene-phthalide; (Z)-4,5-dihydro-6,7-cis-dihydroxy-3-butylidene-phthalide; senkyunolide K; senkyunolide L; senkyunolide M; (Z)-6,7-epoxy-ligustilide; 3,6,7-trihydroxy-4,5,6,7-tetrahydro-3-butyl-phthalide; neocnidilide; 3-n-butyl-3-hydroxyl-4,5,6,7-tetrahydro-6,7-dihydroxyphthalide; (Z,Z')-diligustilide; (Z)-6,8',7,3'-diligustilide; wallichilide; (Z')-3,8-dihydro-6,6_{'},7,3' a-diligustilide; chuanxingzine; tetramethyl-pyrazine; L-isoleucine-L-valine anhydride; adenine; L-valine-L-valine anhydride; trimethylamine; perlolyrine; ferulic acid; sedanonic acid; vanillic acid; caffeic acid; protocatechuic acid; linolenic acid; chrysophanol; methyl phenylacetate; sedanoic acid lactone; methyl pentadecanoate; ethyl p entadecanoate; ethyl heptadecanoate; ethyl isohepta-decanoate; ethyl octadecanoate; ethyl isoctadecanoate; methyl linolenate; lactone; asafetida acid; vanillin; bis-5,5'-formylfurperye ether; 5-hydroxymethyl-6-endo-3'-methoxy-4'-hydroxyphenyl-8-oxa-abicyclo(3,2,1)-oct-3-en-2-one; spathulenol; β-sitosterol; vitamin A; arasaponin A; paeonol; paeonoside; apiopaeonoside; paeconol; paeonolide (paeconol, arabian sugar and paeoniflorin); paeoniflorin; ozypaeoniflorin; benzoylpaeoniflorin; benzoyl-oxypaeoniflorin; gallic acid; 1,2,3,4,6-pentagalloylglucose; hirudin, heparin; rhynchophylline; isorhynchophylline; corynoxeine; isocorynoxeine; corynantheine; dihydrocorynantheine; hirsutine; hirsuteine; heter-Gouteng alkali; katsutoxin; secaline; lycine; cow sulfonic acid; cetylic acid; octadecanoic acid; cholesterin; lecithin; β-Asarone (cis-form); asaryl-aether; Caryophyllene; α-Humulene; Sekishone; 1-allyl-2,4,5-trimethyl-oxy-benzene; β-Asarone; asarone; cis-methylisoengenol; trans-methylisoeugenol; shyobunone; isoshyobuncne; epishyobunone; linalool; calamenene; β-gurjunene; δ-cadinene; calamendiol; isocalamendiol; preisocalamendiol; acoronene; acorone; acoragermacrone; acolamone; isoacolamone; caryophyllene; cis-metylisoeugenol; onjisaponin A, onjisaponin B, onjisaponin C, onjisaponin D, onjisaponin E, onjisaponin F, onjisaponin G; tenuifolin; 2-beta-2,7-dihydroxy-2,3-carboxyoleanolic acid 3-beta-O-glucoside; tenuigenin A; tenuigenin B; Saponin; onjixanthone; 1,6-dihydroxy-3,7-dimethoxy xanthone; 1-hydroxy-3, 6,7-trimethoxy xanthone; 5-anhydro-D-sorbitol; N-acetyl-D-glucosamine; 3,4,5-trimethoxy-cinnamic acid; polygalitol; tenuidine; bilirubin; biliverdin; cholic acid; chenodeoxycholic acid; lithocholic acid; stero-cholic acid; conjugated bile acid; cholestrol; mucigen; carotin; SMC-S; SMC-F; cow cholalic powder; cholalin; pig deoxygenated cholalin; cow sulfonic acid; cholesterin; Dalbergin; Nordalbergin; Isodalbergin; o-Methyldalbergin; Dalbergenone and Dalbergichromene, analogues of said selected compounds and any pharmaceutically acceptable salts thereof.

## Claims

1. A product comprising as a combined preparation:
(i) a composition which comprises the following ingredients: Radix Astragali root (Membranous Milkvetch root or Huang Qi), Radix et Rhizoma Salviae Miltiorrhizae root (Red Sage root or Dan Shen), Radix Paeoniae Rubra root (Red Peony root or Chi Shao), rhizome of Ligusticum Chuanxiong (Chuan Xiong); and
(ii) an agent used in Western medicine (i.e. in any form of mainstream medicine or Western medicine) for the treatment of stroke, for simultaneous, separate or sequential use in the treatment of a patient with a cerebral stroke, heart stroke, neurodegenerative diseases, brain trauma, nervous system trauma or conditions related to neuroplasticity.

2. The product of claim 1, wherein (i) and (ii) are present in a single formulation or as separate formulations.

3. The product of claim 1 or claim 2, wherein the Western medicine is selected from the group consisting of: antiplatelet agents and anticoagulant agents typically used in secondary stroke prevention and neuroprotectants typically used in improving recovery potential in the acute phase of stroke.

4. The product of claim 3, wherein the Western medicine is an antiplatelet agent.

5. The product of claim 3, wherein the Western medicine is an anticoagulant agent.

6. The product of claim 5, wherein the antiplatelet agent is aspirin.
